(19) Europäisches Patentamt
European Patent Office
Office européen des brevets

(11) Publication number : **0 345 856 B1**

# (12) EUROPEAN PATENT SPECIFICATION

(45) Date of publication of patent specification : **05.08.92 Bulletin 92/32**

(51) Int. Cl.$^5$ : **B01J 21/06,** C07D 301/19

(21) Application number : **89201344.2**

(22) Date of filing : **25.05.89**

## (54) A process for the preparation of an oxirane compound.

(30) Priority : **08.06.88 GB 8813484**

(43) Date of publication of application : **13.12.89 Bulletin 89/50**

(45) Publication of the grant of the patent : **05.08.92 Bulletin 92/32**

(84) Designated Contracting States : **BE DE FR GB IT NL**

(56) References cited :
**EP-A- 0 129 814**
**GB-A- 1 602 460**
**US-A- 4 021 454**
**US-A- 4 367 342**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Joustra, Annie Hendrika**
**Badhuisweg 3**
**NL-1031 CM Amsterdam (NL)**
Inventor : **De Bruijn, Wouter**
**Carel van Bylandtlaan 30**
**NL-2596 HR The Hague (NL)**
Inventor : **Drent, Eit**
**Badhuisweg 3**
**NL-1031 CM Amsterdam (NL)**
Inventor : **Reman, Willem George**
**Badhuisweg 3**
**NL-1031 CM Amsterdam (NL)**

**EP 0 345 856 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of an oxirane compound and to oxirane compounds prepared by this process.

It is known to convert olefinic compounds into the corresponding oxirane compounds in that the olefinic compounds are reacted with organic hydroperoxides according to the following general equation:

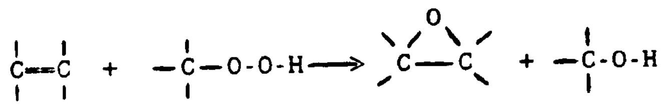

olefinic group    hydroperoxide group    oxirane group    hydroxyl group

From U.K. patent specification 1,249,079 it is further known that the above type of reaction is advantageously carried out with a heterogeneous catalyst which is essentially insoluble in the reaction mixture, said catalyst comprising titanium in chemical combination with a solid silica and/or inorganic silicate.

The reaction is of special importance for the simultaneous preparation of an epoxide of a substituted or unsubstituted alkene having three or four carbon atoms and of a styrene derivative in which process an alkene is reacted with an alpha-arylalkyl hydroperoxide. Epichlorohydrin can be prepared from allylchloride. Propylene oxide can be prepared from propylene.

A well-known sequence of reactions wherein the above-mentioned reaction forms a part, is:

step I

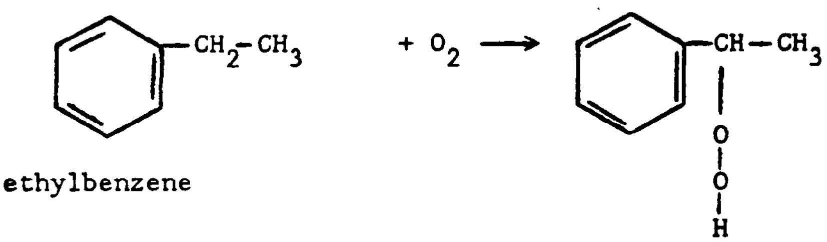

ethylbenzene

ethylbenzenehydroperoxide

step II:

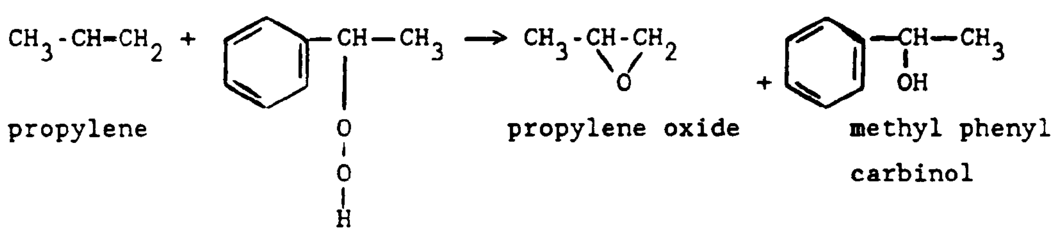

propylene

propylene oxide

methyl phenyl carbinol

step III:

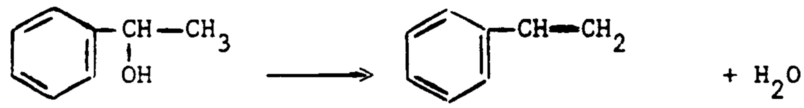

styrene

Another reaction is for example:

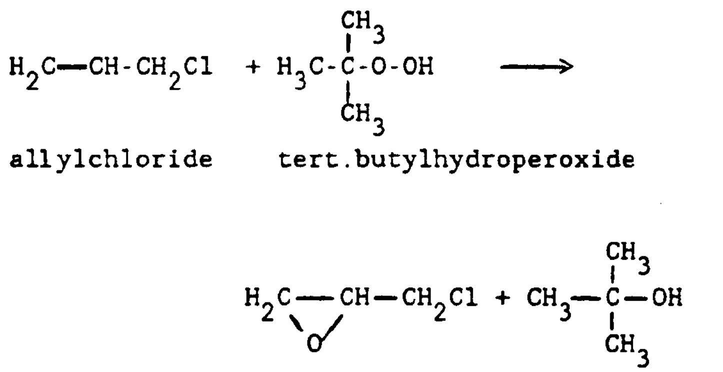

allylchloride tert.butylhydroperoxide

epichlorohydrin tert.butanol.

Applicant has now surprisingly found that in the preparation of the oxirane compound a much more active catalyst is used.

The invention relates to a process for the preparation of an oxirane compound by reacting an olefinic compound with an organic hydroperoxide by the use of heterogeneous catalyst, which is essentially insoluble in the reaction mixture, said catalyst comprising titanium in chemical combination with a solid silica and/or inorganic silicate characterized in that a catalyst is used obtainable by

a) impregnating the silicium compound with a stream of gaseous titanium tetrachloride,

b) calcining the obtained reaction product of step a), and

c) hydrolysis of the product of step b).

Preferably the preparation of the catalyst is performed in the presence of an inert gas, for example nitrogen which is inert under the reaction conditions. Other inert gases are for example carbon dioxide, argon, neon and helium. The inert gas has also the function of a carrier for the gaseous titanium tetrachloride. The titanium tetrachloride may react at rather low temperatures, but because of its low partial pressure the reaction will not be so fast. Therefore higher temperatures, above 130 °C are preferred.

Impregnation of the silicium compound with vaporized titanium tetrachloride proceeds smoothly at for example 180 °C. By a number of thermocouples during the impregnation in the silica or the silicate the temperature is measured and the reaction held under control. Sometime after the breakthrough of the gaseous titanium tetrachloride the supply of it is stopped.

Although the literature is not consistent how far the hydroxyl groups of the silicium compound have reacted with the titanium tetrachloride, it is a fact that the reaction product of step a) still contains to much chlorine in relation to titanium. Calcining the reaction product of step a) removes hydrogen chloride. Preferred calcination temperatures range from 400 °C to 900 °C. A more preferred temperature range is from 500 °C to 700 °C.

Without being bound to any theory, it is believed that the titanium tetrachloride reacts as follows with the silica compound:

I 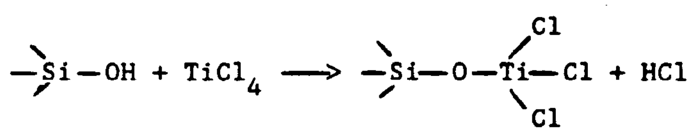

or

II 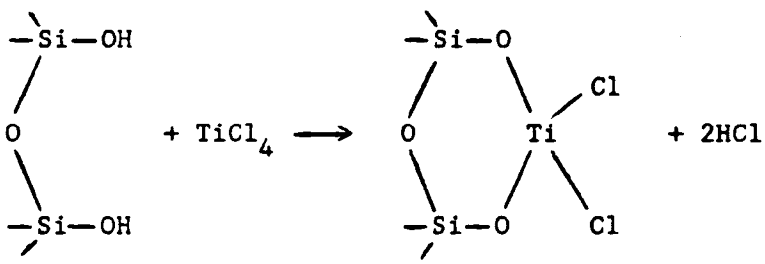

or

III 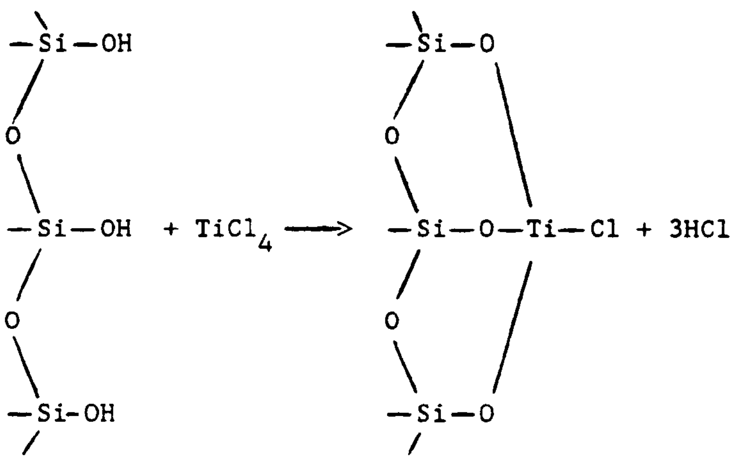

Depending upon the reaction circumstances and the surface of the silica, the product can be a mixture of products of the reactions I, II and III. It is believed that after calcination the stage III is substantially completely reached.

The remaining Ti-Cl bond is then (after calcining) ready to be hydrolyzed. Hydrolyzation may be effected with steam at a temperature preferably in the range of from 150 °C to 400 °C, although higher temperatures are not excluded.

The prepared catalyst may be contacted with a silylating agent, before being used. Silylating agents are, for example, organosilanes, organosilylamines and organosilazanes. Preferred are tetra-substituted silanes with 1-3 hydrocarbyl substituents, such as chloro trimethyl silane, dichloro dimethyl silane, chloro bromo dimethyl silane, nitro trimethyl silane, chloro triethyl silane, iodo dimethyl butyl silane and chloro dimethyl phenyl silane. A very suitable silylating agent is hexamethyldisilazane having the chemical formula

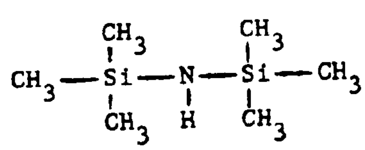

The latter compound is of special importance, since it reacts at relatively low temperatures, namely between 100 °C and 300 °C. Generally the silylating agents react in gaseous phase. The organic hydroperoxide, represented by the general formula R-O-O-H, in which R is a monovalent hydrocarbyl group, reacts with an olefinic compound to an oxirane compound and a compound R-OH.

Preferably, the group R has from 3 to 20 carbon atoms. Most preferably, it is a hydrocarbyl group, in particular a secondary or tertiary alkyl or aralkyl group, having from 3 to 10 carbon atoms. Especially preferred among these groups are the tertiary alkyl and secondary or tertiary aralkyl groups, including, e.g., tertiary butyl, tertiary pentyl, cyclopentyl, 1-phenylethyl-1, 2-phenylpropyl-2 and the various tetralinyl radicals which originate by elimination of a hydrogen atom from the aliphatic side-chain of the tetralin molecule.

Aralkyl hydroperoxide, wherein the hydroperoxy group is linked to that carbon atom of an alkyl side-chain which is attached directly to an aromatic ring, including 1-phenylethyl-1-hydroperoxide and 2-phenylpropyl-2-hydroperoxide, are often called after the corresponding hydrocarbons, e.g. ethyl benzene hydroperoxide and cumene hydroperoxide. It will be appreciated that, when using ethylbenzene hydroperoxide, the resulting hydroxyl compound is 1-phenylethanol-1, also to be called methyl phenyl carbinol, which may be dehydrated to styrene, and that, when using cumene hydroperoxide, the resulting hydroxyl compound is 2-phenylpropanol-2, also to be called dimethyl phenyl carbinol, which may be dehydrated to alpha-methylstyrene. Of course, both styrene and alpha-methylstyrene are industrially useful products.

Tertiary amylenes, which are useful as isoprene precursors, may be obtained by dehydration of the alcohol, which is formed when using tertiary pentyl hydroperoxide.

The organic hydroperoxide reactant used as a starting material may be in a dilute or concentrated, purified or unpurified condition.

In principle, any organic compound having at least one olefinic double bond may be reacted with an organic hydroperoxide. The compound may be acyclic, monocyclic, bicyclic or polycyclic and they may be mono-olefinic, diolefinic or polyolefinic. If there are more than one olefinic linkages, these may either be conjugated or non-conjugated. Generally preferred are olefinic compounds having from 2 to 60 carbon atoms. Although substituents, which should preferably be relatively stable, may be present, acyclic mono-olefinic hydrocarbons having from 2 to 10 carbon atoms are of particular interest. Such hydrocarbons include, e.g., ethylene, propylene, isobutylene, hexene-3, octene-1 and decene-1. Butadiene may be mentioned as an example of a suitable diolefinic hydrocarbon. Substituents, if present, may, e.g., be halogen atoms or comprise atoms of oxygen, sulphur and nitrogen together with atoms of hydrogen and/or carbon. Of particular interest are olefinically unsaturated alcohols, and halogen-substituted olefinically unsaturated hydrocarbons, including, e.g., allyl alcohol, crotyl alcohol and allyl chloride. Particularly preferred are alkenes having from 3 to 40 carbon atoms, which may or may not be substituted with a hydroxy or a halogen atom.

Oxirane compounds are materials of established utility and many are chemicals of commerce, in particular olefin oxides such as, e.g., ethylene oxide and propylene oxide. Propylene oxide may be converted into useful polymeric products by polymerization or copolymerization. Of commercial interest is also epichlorohydrin which may be obtained from allyl chloride and may, if so desired, be converted into glycerol. Of course, glycerol may also be made from the oxirane compound which is formed when starting from allyl alcohol.

Now, the present invention relates to the use of a specifically prepared catalyst composition which is essentially insoluble in the epoxidation reaction mixture, thus providing a heterogeneous system. It has surprisingly been found that the catalyst composition of the invention is very active, and leads to high conversions of the organic hydroperoxide(s) introduced with high selectivities to the oxirane compound(s) concerned, these selectivities being defined as the molar ratios of oxirane compound(s) formed to organic hydroperoxide(s) converted.

In general, the epoxidation reaction is conducted in the liquid phase using solvents and/or diluents which are liquid at reaction temperature and pressure, and substantially inert to the reactants as well as to the products. The presence of reaction materials such as, e.g., water is desirably avoided. A substantial part of the solvent may consist of materials present in the hydroperoxide solution employed. Preferred solvents further to be added are mononuclear aromatic compounds, e.g., benzene, toluene, chlorobenzene, bromobenzene, orthodichlorobenzene, and alkanes, e.g. octane, decane and dodecane. However, an excess amount of the olefinic reactant may also serve as a solvent together with the solvent material introduced together with the organic hydroperoxide, so that no addition of any further solvents is needed. In most instances, however, added solvent is used. The total amount of solvent material may be up to 20 moles per mole of the hydroperoxide.

The epoxidation reaction, generally, proceeds at moderate temperatures and pressures, in particular at temperatures in the range of from 0 °C to 200 °C, the range from 25 °C to 200 °C being preferred. The precise pressure is not critical so long as it suffices to maintain the reaction mixture in a liquid condition. Atmospheric pressure may be satisfactory. In general, pressures are suitably in the range of from 100 to 10000 kPa.

At the conclusion of the epoxidation reaction, the liquid mixture comprising the desired products may easily be separated from the solid catalyst material. The liquid mixture may then be worked up using any suitable conventional methods, including, e.g., fractional distillation, selective extraction and filtration. The solvent, the catalyst and any unreacted olefin or hydroperoxide may be recycled for further utilization. The process of the invention can successfully be carried out with the catalyst in the form of a slurry, of a moving bed or a fluidized bed. However, a fixed catalyst bed is preferred for large-scale industrial operation. The process may be carried out in a batch-wise manner, semi-continuously or continuously. The liquid containing the reactants may then

be passed through the catalyst bed, so that the effluent from the reaction zone is entirely or at any rate practically free from catalyst material.

The specifically prepared catalyst employed according to the present invention comprises titanium in chemical combination with a solid silica and/or inorganic silicate. The titanium will during the reaction assume the tetravalent state, and is preferably combined with the solid silica and/or inorganic silicate in that oxidation state. The proportion of titanium in the catalyst composition can be varied, but proportions of at least 0.1% by weight of titanium calculated as the dioxide, are as a rule very satisfactory, proportions of from 0.2% to 50% by weight, calculated on the same basis, being preferred. However, still larger proportions of titanium can be used.

Suitable solid silicas and/or inorganic silicates contain at least 50%, but, preferably, at least 75% and, most preferably, at least 90% by weight of silicon, calculated as the dioxide, that is silica. Suitably, the solid silicas and/or inorganic silicates are further characterized by having a relatively large specific surface area, in particular a specific surface area of at least 1 $m^2/g$. Preferably, the specific surface area is in the range of from 25 to 800 $m^2/g$.

Suitable is, e.g., synthetic, porous silica in a relatively dense, close-packed form, consisting of particles of amorphous silica flocculated or linked together, including silica gel and precipitated silica. The preparation and properties of such materials are described by R.G. Iler in his book "The Colloid Chemistry of Silica and Silicates", Cornell University Press, New York, 1955, Chapter VI and in U.S. patent specification 2,657,149. Among the silica gels which are available commercially, those with a specific surface area in the range of from 25 to 700 $m^2/g$ and a pore volume in the range of from 0.3 to 1.3 ml/g, and which consist to at least 99% by weight of silica, are generally the most suitable.

Also suitable are, however, synthetic silica powders consisting of particles of amorphous silica flocculated in openpacked, readily disintegrated, loosely-knit aggregates, such as, e.g., fumed pyrogenic silica obtained by the combustion of hydrogen and oxygen with silicon tetrachloride or tetrafluoride. Such products are commercially produced and sold by various companies, including, e.g., Cabot Corportion ("Cab-O-Sil") and Degussa ("Aerosil"). (The words "Degussa" and "Aerosil" are registered Trade Marks). Among these products those with a specific surface area in the range of from 50 to 400 $m^2/g$ and which to at least 99% consist of silica, are generally the most suitable.

Furthermore, suitable solid silicas and/or inorganic silicates include the crystalline alumino-silicates which are known in the art as molecular sieves, as well as naturally-occurring crystalline mineral silicates, including, e.g., asbestos minerals, e.g., serpentine (hydrous magnesium silicates); clay minerals, e.g., hectorite (magnesium lithium silicate), kaolins and bentonites, and micaceous minerals, e.g., phlogopite (potassium magnesium aluminium silicate) and vermiculite (a hydrous magnesium silicate). Preferred are, however, synthetic amorphous solid silicas and/or inorganic silicates, in particular those which essentially consists of substantially pure silica, e.g., at least 95% silica.

It may be advantageous to include promoters in the catalysts to be used according to the invention, in particular, compounds of alkaline earth metals, including, e.g., magnesium, calcium, strontium and barium. Preferred are the oxides and such compounds as are readily convertible into oxides. For effecting this conversion a pretreatment of the initially prepared catalyst composition prior to its use may be desirable. The proportions of the promoter or promoters are not critical, but proportions exceeding 10% by weight, calculated as metal on the catalyst support, are not as a rule required. The inclusion of promoters is especially advantageous when employing solid silicas and/or inorganic silicates with strongly acidic sites, e.g., when the intrinsic acidity is less than -3. The intrinsic acidity, generally represented by pKa, is determined by titration of the material concerned with an appropriate base in the presence of dye indicators, as disclosed, for example, in U.S. patent specification No. 2,868,688.

The catalyst may be employed in any convenient physical form, for example, in the form of a powder, as well as of flakes, spheres or pellets.

## EXAMPLE

### Preparation of the titanium catalyst

120 g of dried silica was charged into a quartz reactor and heated in a nitrogen stream of 20 l per hour to a temperature of 180 °C.

Titanium tetrachloride was heated in a 3-neck flask to the boiling point of $TiCl_4$ and the resulting $TiCl_4$ vapour was distilled over the silica bed, using nitrogen as a carrier gas for $TiCl_4$.

When the titanium tetrachloride vapour came into contact with the silica, at the spot of the reaction the temperature of the silica bed increased with 20 °C. This feature was used to detect the moment that the reaction zone reached the end of the bed and a breakthrough of $TiCl_4$ occurred. During 1.5 hour 220 ml $TiCl_4$ was con-

ducted over the silica bed. After termination of the $TiCl_4$ distillation, dry nitrogen was led over the silica bed for 0.5 hour at 180 °C and at a rate of 20 l per hour.

The impregnated silica was then heated in a nitrogen atmosphere to 600 °C (at a rate of 50 °C/h) and calcined for 4 hours at 600 °C. The calcined silica/titania catalyst was cooled to 300 °C and steam was added to the circulating nitrogen which flowed at a rate of 20 l per hour over the catalyst. Steam treatment was carried out for two hours. The amount of steam was 12% by weight of the amount of nitrogen led over the catalyst.

The reactor was then cooled to 200 °C in a stream of dry nitrogen and hexamethyldisilazane was conducted over the catalyst bed for two hours using dry nitrogen as a carrier gas. An exotherm of 30 °C was observed, indicating a reaction of hexamethyldisilazane with hydroxyl groups in the catalyst. Excess of hexamethyldisilazane was stripped with nitrogen. Without being bound any theory, it is believed that the silylation reaction proceeds as follows:

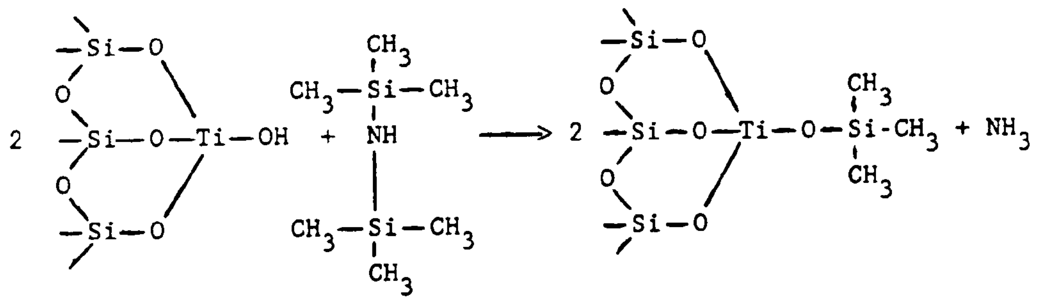

Preparation of oxirane compound

The catalyst performance was tested in a tubular reactor which contained a catalyst as prepared above.

The epoxidation of propene was carried out with ethylbenzene hydroperoxide. A reaction mixture of about 6 mol propene per mol ethylbenzene hydroperoxide in ethylbenzene was continuously led through the reactor, with a liquid hourly space velocity of 5.58 $h^{-1}$. At 200 hours of operation the following conditions applied: Ethylbenzene hydroperoxide conversion was kept at 95.3 mol% by regulation of the temperature. The propene feed rate was 47,2 g per hour. The ethylbenzene hydroperoxide/ethylbenzene feed rate (27% mol/mol) was 96.6 g per hour. The propene/ethylbenzene hydroperoxide molar ratio was 5.98. The reaction temperature was 60 °C. The selectivity (to propylene oxide) was 95,9%.

EXAMPLE (Comparative)

A titanium silica catalyst, prepared by impregnation of silica with a solution of tetraisopropylorthotitanate, complexed with acetylacetone in isopropylalcohol and by silylation with hexamethyldisilazane, was also tested. Under comparable conditions after 200 hours of operation only the temperature was different, viz. the reaction temperature was 90 °C.

Since the activation energy for the reaction is 50 kJ/mol and since a temperature difference of 30 °C was measured the conclusion is that the catalyst used in the process according to our invention is 4.5 times as active as the catalyst used in the prior art process.

## Claims

1. A process for the preparation of an oxirane compound by reacting an olefinic compound with an organic hydroperoxide by the use of a heterogeneous catalyst, which is essentially insoluble in the reaction mixture, said catalyst comprising, titanium in chemical combination with a solid silica and/or inorganic silicate characterized in that a catalyst is used obtainable by

a) impregnating the silicium compound with a stream of gaseous titanium tetrachloride,

b) calcining the obtained reaction product of step a), and

c) hydrolysis of the product of step b).

2. A process as claimed in claim 1 wherein the catalyst before use is brought into contact with a silylating agent at elevated temperature.

3. A process as claimed in claim 1 or 2 wherein the preparation of the catalyst is performed in the presence of an inert gas.

4. A process as claimed in claim 3 wherein the inert gas is nitrogen.

5. A process as claimed in one or more of the claims 1-4 wherein the temperature in step a) is above 130 °C.

6. A process as claimed in one or more of the claims 1-5, wherein the calcination temperature is between 400 °C and 900 °C, preferably between 500 °C and 700 °C.

7. A process as claimed in one or more of the claims 1-6 wherein the hydrolysis takes place with steam at a temperature of between 150 °C and 400 °C.

8. A process as claimed in claim 2 wherein the organic silylating agent is hexamethyldisilazane.

9. A process as claimed in claim 2 or 8 wherein the temperature of silylation is between 100 °C and 425 °C.

## Patentansprüche

1. Verfahren zur Herstellung einer Oxiranverbindung durch Umsetzung einer olefinischen Verbindung mit einem organischen Hydroperoxid unter Verwendung eines heterogenen Katalysators, der im wesentlichen in dem Reaktionsgemisch unlöslich ist, wobei der Katalysator umfaßt titan in chemischer Kombination mit einer festen Rieselsäure und/oder einem anorganischen Silicat, dadurch gekennzeichnet, daß ein Katalysator verwendet wird, erhältlich durch

a) Imprägnieren der Siliciumverbindung mit einem Strom von gasförmigem Titantetrachlorid,

b) Calcinieren des in Stufe a) erhaltenen Reaktionsproduktes und

c) Hydrolyse des Produktes der Stufe b).

2. Verfahren nach Anspruch 1, wobei der Katalysator vor der Verwendung mit einem Silylierungsmittel bei erhöhter Temperatur zusammensebracht wird.

3. Verfahren nach Anspruch 1 oder 2, wobei die Herstellung des Katalysators in Gegenwart eines Inertgases durchgeführt wird.

4. Verfahren nach Anspruch 3, wobei das Inertgas Stickstoff ist.

5. Verfahren nach einem der Ansprüche 1 bis 4, wobei die Temperatur in Stufe a) über 130°C liegt.

6. Verfahren nach einem oder mehreren der Ansprüche 1 bis 5, wobei die Calcinierungstemperatur zwischen 400°C und 900°, vorzugsweise zwischen 500°C und 700°C, liegt.

7. Verfahren nach einem oder mehreren der Ansprüche 1 bis 6, wobei die Hydrolyse mit Dampf bei einer temperatur zwischen 150°C und 400°C stattfindet.

8. Verfahren nach Anspruch 2, wobei das organische Silylierungsmittel Hexamethyldisilazan ist.

9. Verfahren nach Anspruch 2 oder 8, wobei die temperatur der Silylierung zwischen 100°C und 425°C liegt.

## Revendications

1. Un procédé pour la préparation d'un composé du type oxiranne en faisant réagir un composé oléfinique avec un hydroperoxyde organique avec utilisation d'un catalyseur hétérogène, qui est essentiellement insoluble dans le mélange réactionnel, ce catalyseur comprenant du titane en combinaison chimique avec une silice et/ou un silicate inorganique solides, caractérisé en ce qu'on utilise un catalyseur pouvant être obtenu par :

a) imprégnation du composé du silicium avec un courant de tétrachlorure de titane gazeux,

b) calcination du produit de réaction obtenu dans l'étape a) et

c) hydrolyse du produit de l'étape b.

2. Un procédé selon la revendication 1, dans lequel le catalyseur avant utilisation est mis en contact avec un agent de silylation à température élevée.

3. Un procédé selon la revendication 1 ou 2, dans lequel la préparation du catalyseur est effectuée en présence d'un gaz inerte.

4. Un procédé selon la revendication 3, dans lequel le gaz inerte est de l'azote.

5. Un procédé selon une ou plusieurs des revendications 1-4, dans lequel la température dans l'étape a) est au-dessus de 130°C.

6. Un procédé selon une ou plusieurs des revendications 1-5, dans lequel la température de calcination est comprise entre 400°C et 900°C, de préférence entre 500°C et 700°C.

7. Un procédé selon une ou plusieurs des revendications 1-6, dans lequel l'hydrolyse a lieu avec de la vapeur d'eau à une température comprise entre 150°C et 400°C.

8. Un procédé selon la revendication 2, dans lequel l'agent de silylation organique est l'hexaméthyldisilazane.

9. Un procédé selon l'une des revendications 2 ou 8, dans lequel la température de silylation est comprise entre 100°C et 425°C.